# EUROPEAN PATENT SPECIFICATION

(11) **EP 0 496 792 B1**
(45) Date of publication and mention of the grant of the patent: **25.01.1995**
(21) Application number: 90915788.5
(22) Date of filing: 16.10.1990
(51) Int. Cl.: A61M 1/34

(54) **TRANSMEMBRANE PRESSURE CONTROLLED FILTRATION SYSTEM**
DURCH TRANSMEMBRANEN DRUCKREGULIERTES FILTRATIONSSYSTEM
SYSTEME DE FILTRATION A PRESSION REGLEE A TRAVERS UNE MEMBRANE

(30) Priority: 17.10.1989 GB 8923376
(43) Date of publication of application: 05.08.1992
(73) Proprietor: BIO-FLO LIMITED, Glasgow G41 1ST (GB)
(72) Inventor: HOOD, Robert, Gordon, Paisley, PA2 6HW (GB)
(74) Representative: Naismith, Robert Stewart
(86) International application number: GB9001596
(87) International publication number: WO9105576

(56) References cited:
- EP-A- 0 010 584
- EP-A- 0 240 101
- FR-A- 2 581 316
- GB-A- 2 053 724
- US-A- 3 946 731
- US-A- 4 077 882

## Description

The present invention relates to fluid flow control apparatus for use in separation and filtration systems, and in particular to apparatus for controlling the transmembrane pressure in a filtration device.

Fluid flow control apparatus for use in separation and filtration systems and similar fluid handling systems such as a life support systems should satisfy a number of desirable criteria in addition to being efficient and relatively inexpensive. The constituent parts of the fluid control apparatus should be easily cleaned or sterilised or be disposable as this is important when using biological fluids such as blood. The apparatus should require minimal fluid volume for analysis and should accommodate sensors for monitoring various fluid parameters of the fluid. The apparatus should also permit control of fluid flow rate and pressure to suit specific separation and filtration requirements.

Existing separation and filtration equipment does not fulfil one or more of the above mentioned requirements. In particular, existing equipment does not facilitate sterilisation requiring disassembling of components and such equipment and often requires substantial volumes of fluid for monitoring purposes. These drawbacks are particularly evident when dealing with the treatment of biological fluids, for example, blood in dialysis or life support systems. In addition, control of flow rate and pressure is often complex in existing equipment and further reduces the sterilisability of the equipment. Thus, most existing systems tend to be aseptic rather than sterile, and this is less desirable in a clinical environment.

In many applications it is desirable to control the filtration pressure within the filter unit so as to optimise filtration and at present this is not achieved with existing systems. Furthermore in applications involving filtration of biological fluids it is highly desirable that there should be no contact between the fluid being treated and the monitoring system to avoid the possibility of contamination. This is particularly relevant in life support systems. In other applications such as the isolation of cells and products from fermentation broths and the high value liquids, it is desirable that there is minimal loss of biomass and cell viability.

EP-A-0240101 discloses a membrane filtration device comprising a filter having an inlet for receiving blood to be filtered and an outlet for receiving the outlet fluid from the filter. An arterial pressure sensor measures the inlet pressure to the filter and a venous pressure sensor is associated with the outlet for measuring the pressure of the blood flowing from the filter. A central processor compares the arterial and venous pressures and controls an arterial pump and a venous pump to provide a desired pressure across the filter.

It is among the objects of the present invention to provide a fluid flow control apparatus which obviates and mitigates at least one of the aforementioned problems.

The present invention is defined in appended claim 1.

In one embodiment of the invention, the objects of the invention are achieved by providing apparatus for controlling the transmembrane pressure in which a removable sterilisable or disposable element is provided and in which the pressure of the fluid inlet and fluid outlet of a filter unit are monitored and the pressures are compared and used to control the pressure across the filter unit. This is particularly important in cross-flow filtration which is a procedure whereby the process fluid is recirculated over the membrane surface. In the present case the fluid flow through the filter creates a pressure differential between the inlet and the outlet and the driving force through the membrane is determined by the transmembrane pressure (TMP). The transmembrane pressure is obtained by measuring the inlet and outlet fluid pressures and averaging these values. The pressure of the filtrate has to be substracted from the average value, however, the filtrate pressure is small and is generally taken as zero.

In a preferred embodiment, pressure is measured by bleeding the inlet conduit through an inlet bleed conduit which includes a pressure switch and pressure transducer. The outlet conduit containing the retentate also has a bleed line and a post-membrane pressure transducer is inserted in the second bleed off line. With this arrangement the inlet and outlet pressures can be monitored and the pressure values are averaged in use to control the pressure switch or valve so as to maintain optimal control of the transmembrane pressure and optimise filtration for the particular fluids and membranes used.

In one arrangement, the flow-through arrangement, the pressure transducers are located in the bleed lines which means that the sensors are in contact with the fluid being treated. In another arrangement, the pressure transducers are of the diaphragm type as is the pressure switch so that there is no contact between the fluid being treated and the monitoring and controlling apparatus.

In a preferred arrangement the pressure monitoring and control system is incorporated within a fluid handling system which includes a fixed portion and a sterilisable or disposable portion which is removably engageable with the fixed portion. The removable portion is connectable to a pump and to conduits in the fluid handling system.

The fixed portion is shaped to receive the removable portion, known as the cassette, and the fixed portion has apertures therein for receiving the pressure sensors and the pressure switch. When the cassette is inserted into the fixed portion, the apertures in the cassette and fixed portion are aligned. In the flow-through system the fluid passes between the cassette and the fixed portion through aseptic parts. In contrast, fluid does not pass when the diaphragm sensors are used because the diaphragm pressure sensor in both the cassette and fixed portion are aligned and pressure is monitored via the diaphragms without contacting the fluid being treated.

In the diaphragm sensor arrangement, both pressure transducers in the fixed part contain channels which can be filled with fluid, the pressure of which can be adjusted to calibrate the sensors for the system being monitored.

According to one aspect of the present invention there is provided a membrane filtration device comprising filter means having an inlet for receiving an inlet fluid to be filtered and an outlet for receiving the outlet fluid from said filter means, first pressure monitoring means associated with the inlet for measuring the inlet pressure to said filter means, second pressure monitoring means associated with the outlet for measuring the pressure of the outlet fluid, means for comparing the inlet and outlet pressures measured and flow control means coupled to said first and second pressure monitoring means to provide a comparison signal, the flow control means being responsive to the comparison signal to control the flow of fluid through said filter means characterised in that the device further comprises an inlet bleed conduit for fluid communication between the inlet and outlet, and wherein the control of the flow of fluid through the filter means is achieved by varying the flow of inlet fluid through the inlet bleed conduit, to optimise control of the transmembrane pressure and filtration.

Preferably the fluid flow control means is a pressure switch located in the inlet bleed conduit. Alternatively, the pressure switch may be located in an outlet bleed conduit or may be a valve for restricting flow in the inlet or outlet bleed conduits.

Preferably the pressure monitoring means are disposed in the inlet and outlet bleed conduits so that the fluid flows past the sensors. Alternatively the pressure monitoring means are diaphragm pressure sensors so that there is no contact between the fluid being treated and the pressure monitoring means.

The first and second pressure monitoring means may be coupled to first and second solenoid valves respectively, the outputs of which are combined within a single bleed conduit, the valves being actuatable to purge fluid from the measurement fluid circuit.

The transmembrane pressure controlled filtration system may be used with a fluid flow control apparatus which includes a fixed portion and a removable portion, the fixed portion having pressure sensors and a pressure switch disposed therein and coupled to internal conduits within the fixed portion to facilitate flow-through of the fluid being monitored. The removable portion contains internal conduits which terminate in ports which mate with the corresponding ports in the fixed portion so as to form aseptic ports when the removable portion is inserted into the fixed portion.

Alternatively, the removable portion contains at least two diaphragms each of which is disposed in the surface of the removable portion, one diaphragm being arranged to receive, through internal conduits, fluid from the filter and the output of which is fed to the valve, the other diaphragm being arranged to receive fluid from the pump and to return this fluid to the filter. Disposed in the fixed portion are corresponding diaphragms such that when the removable portion is inserted into the fixed portion, the diaphragms register with each other, the diaphragms on the fixed portion being coupled to respective pressure gauges for monitoring the filter inlet and outlet pressures and coupled to internal conduits which are filled with fluid to calibrate the pressure sensors.

These and other aspects of the invention will become apparent from the following description when taken in combination with the accompanying drawings in which:-
Figure 1 is a perspective and partly exploded view of fluid control apparatus;
Figure 2 is a view of the apparatus of Fig. 1 taken in the direction of arrow 2;
Figure 3 is a diagrammatic representation of an embodiment of a transmembrane pressure monitoring system in accordance with the present invention;
Figures 4 a to e are partly sectioned plan, end and elevational views of a removable cassette in accordance with an embodiment of the invention in which the cassette contains diaphragm pressure transducers;
Figures 5 a - d are plan, end and elevational views of the fixed part of the flow control system for receiving the removable cassette and which is modified to receive corresponding diaphragm pressure sensors and a pressure switch:
Figure 6 is an enlarged side view of a diaphragm sensor mounted in the removable cassette, and
Figure 7 is a graph of pressure against time for a fitted back-wash procedure using the system of Figs. 1 to 6 incorporated in a downstream processing unit.

The fluid flow control apparatus 10 consists of two parts; a first main body portion, generally indicated by reference numeral 12, which remains fixed in the BIO 2000 control unit and a removable cassette element 14. The main body portion 12 has two parts; a cassette guide 16 and a separate valve control part 18. The guide 16 and valve 18 are spaced apart to define a channel 20 for slidably receiving the removable cassette element 14 in a close fitting arrangement.

The guide 16 has aseptic ports 22a, b disposed on its inner surface 24 for registering with like ports 23a, b disposed on the outer surface 25 of cassette element 14. In the assembled condition pressure sensors 67, 84 and pressure switch 65 are securely fastened in the top of the guide 16 to monitor these fluid parameters. Pressure sensitive sensors 67, 84 measure the inlet and outlet pressures as will be described.

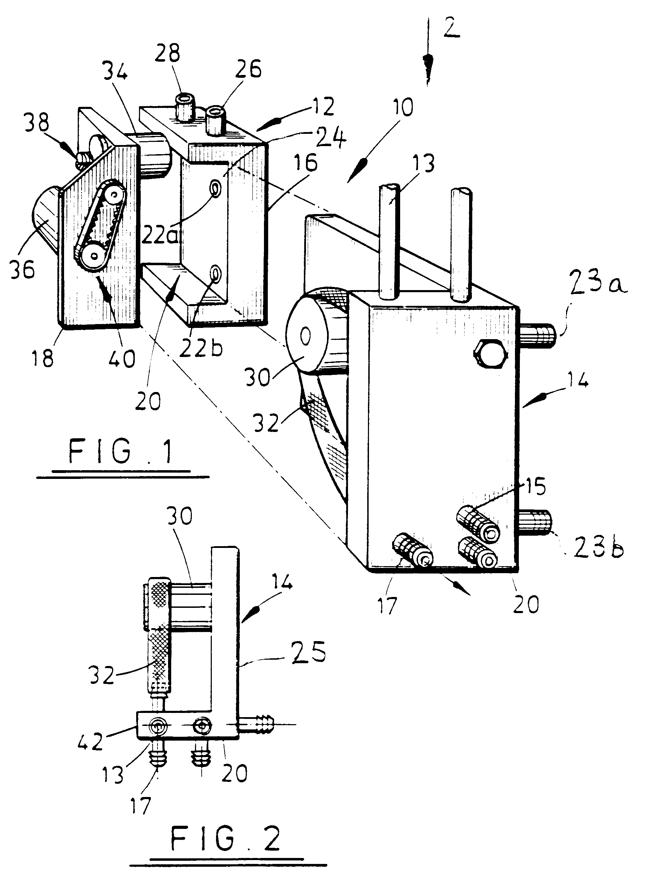

The cassette element 14 is generally L-shaped in plan and consists of a machined block of medical grade sterilisable stainless steel and is of sufficient thickness to accommodate fluid flow channels as will be fully explained later. The cassette element 14 contains the apparatus inlet 15 and various other inlets and outlets as will also be explained. The cassette also includes an element which is part of the control valve 18. This is a fixed cylindrical boss 30 around which is located a flexible conduit 32 which connects the conduit 13 tube with the unit outlet tube 17. The main part of control valve 18 includes a rotable cam 34 coupled to an electric motor 36 via gears 38 and a chain and sprocket drive 40. When the cassette element 14 is in an assembled position as in Fig. 1 or 2, the electric motor is operable by a central control unit, not shown within apparatus 10, to control the position of cam 34 so that the distance between the cam 34 and boss 30 is controlled to define a 'nip' on tube 32. This nip is used to create the back pressure in the separation element or filter to suit the filtration requirements of the blood.

Reference is now made to Figures 1, 2 and 3 of the drawings which depicts a fluid circuit generally indicated by reference numeral 50 for use with a BIO 2000 filtration system (Bio-flow Ltd., U.K.). In the system shown in Figures 1, 2 and 3 a fluid conduit 52 has an inlet for receiving fluid to be treated. A three lobe roller pump 54 forces the fluid along conduit 52 and through an inlet 58 to a hollow fibre filter unit generally shown by reference numeral 60. A bleed line 62a is taken from the inlet conduit 64 so that the inlet fluid can be monitored by various sensors. In the example shown, the pressure transducer 65 and pressure switch 67 are located in the bleed line 62 for measuring the pressure parameters of the inlet fluid. The bleed line is fed to a solenoid valve 66, the output of which is connected to a conduit 70a which, in turn, is coupled via outlet 72 to the system being treated.

The filter unit has a filter output 74 which feeds outlet conduit 76 which passes through a pressure control valve, not shown in the interest of clarity, and the output from the valve constitutes the return fluid which is fed back to the source, in this example the vascular system of a patient. The outlet conduit has a bleed line 78 which is connected to a second solenoid valve 80 which has an output 82 which feeds into input conduit 70 as described above. A pressure transducer 84 is located in the bleed line 78 for measuring the pressure of the filter output fluid. It will be understood that conduits 62a,78a and 70b are located in the removable portion 14 or cassette of the BIO 2000 filtration system when these conduits are coupled via aseptic ports 63, 77 and 81 to conduits 62b, 78b and 70a respectively which are located in the fixed portion 12.

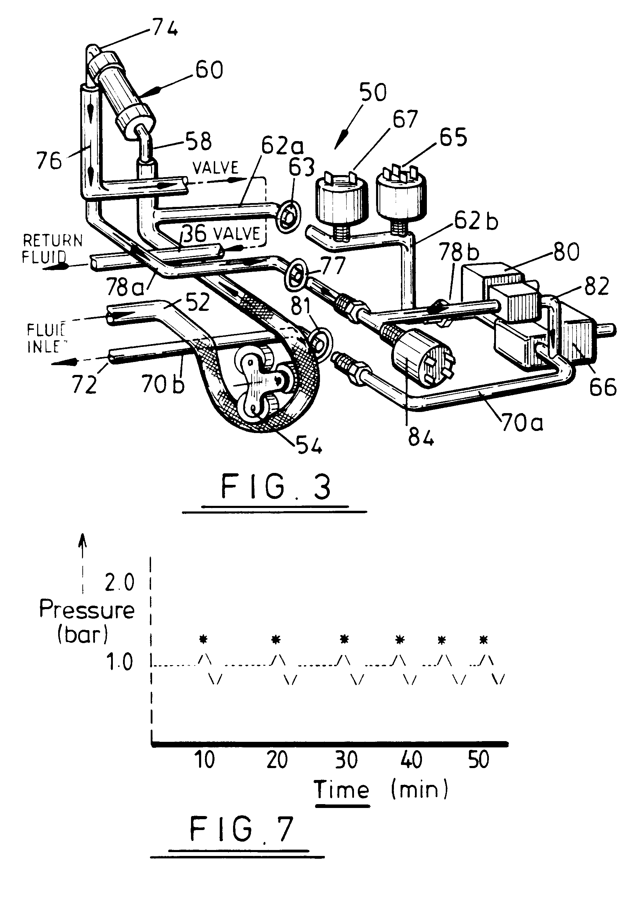

In the arrangement shown in Figure 3, the pressure switch 67 and pressure sensors 65 and 84 are located in line so that the fluid flows through and is in contact with the sensors.

With this arrangement, transmembrane pressure measurement (TMP) is achieved by monitoring the inlet pressure with transducer 24 and the outlet pressure with transducer 84. The transmembrane pressure is calculated at the average of the inlet and outlet pressures (Po + Pi/2) less the filtrate pressure (P_{f}). However, filtrate or permeate pressure is low and is generally taken as zero so that the average transmembrane pressure is deemed to be the average of the inlet and outlet pressures. The measured pressures are processed in a routine manner to provide the average pressure used to control pressure switch 67 to vary the flow through the solenoid or to affect the back pressure of the system or can be used controlling the valve to vary the pressure in the filter outlet conduit by varying the distance between the cam and boss and thus affect the transmembrane pressure. With this arrangement, control of the transmembrane pressure is optimised to minimise effects of gel polarisation and the like which is a process which is known to slow down filtration.

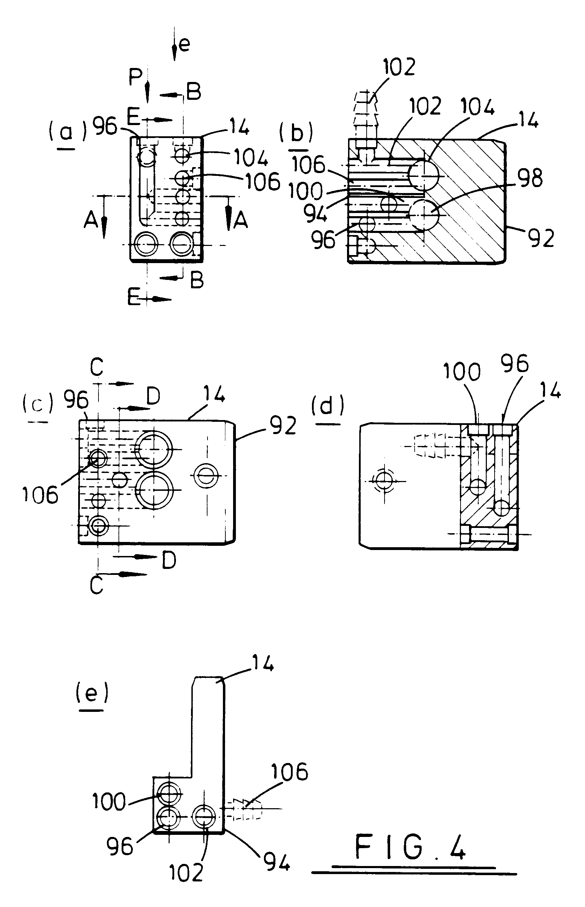

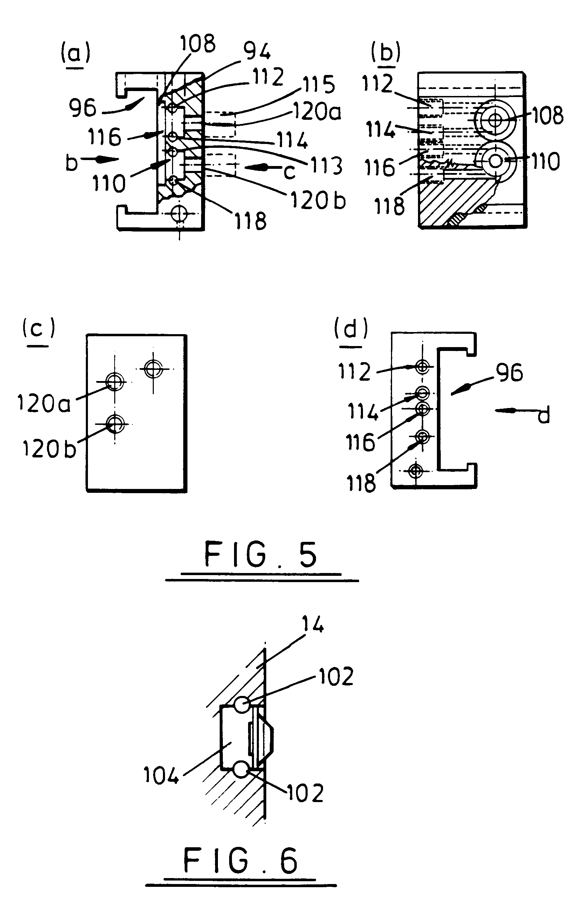

Reference is now made to Figures 4a to e and 5a to d of the drawings which depicts the removable and fixed part respectively of the fluid flow control apparatus BIO 2000 filtration system. Figures 4a to e show various views of the removable cassette which is adapted to slide into the fixed portion shown in Figures 5a to d. In this embodiment the system is generally similar to that shown in Figure 1, 2 and 3 except that pressure is measured using diaphragm pressure system as opposed to a flow-through system as will be now explained. This means that the fluid being filtered does not contact the monitoring system which has important implications in maintaining the sterility and the integrity of the fluid being treated.

As best seen in Figs. 4a to e, the removable cassette 14 is generally L-shaped in plan (Fig. 4e). The middle portion has a leading end 92 which is shaped to be inserted into the fixed portion 94 to be received by channel 96. The fluid connections to the removable cassette are therefore principally at the trailing end 94. In the embodiment shown in Fig. 3 fluid is pumped in through inlet 96 as best seen in Figs. 4a and 4b whereupon it enters the diaphragm chamber 98 fitted with a diaphragm (not shown in the interest of clarity) and then exits along internal conduit 100. The filter output is fed in through conduit 102 to diaphragm chamber 104 and out through internal conduit 106.

Reference is now made to Fig. 5 of the drawings which depicts the fixed part 12 of the cassette unit which is machined to receive the removable portion 14 within channel 96 in a sliding manner so that the diaphragms 98,104 lie in register with diaphragms 108,110 of the fixed portion respectively. Diaphragm chamber 108 is connected to inlet and outlet conduits 112,114 and diaphragm chamber 110 is connected to conduits 116,118 as best seen in Figs. 5a and 5b. Diaphragm chambers 108,110 are dimensioned to receive a diaphragm similar to that in chambers 98 and 104 as best seen in Fig. 6 and chambers 108 and 110 communicate with openings 120a and 120b into which pressure transducers may be fitted. Internal conduit 112 communicates with opening 120 into which a pressure switch may be disposed so that the cassette system is arranged to facilitate transmembrane pressure monitoring as described in the first embodiment.

Reference is made to Fig. 6 which depicts an enlarged view of a section through diaphragm aperture 104 shown in Fig. 4b. It will be seen that a diaphragm sensor is fitted as shown and projects slightly proud of the surface of the cassette 14. The diaphragms have an impervious silastic membrane to prevent fluid leakage. When the removable cassette 14 is inserted into channel 96 the diaphragms are aligned and contact each other so as to transmit a force from one membrane to the other. When the system is set up it is important that it is calibrated so that changes in pressure are transmitted correctly through the membrane for accurate measurement. This is achieved using the arrangement shown in Figs. 5a and b in which the diaphragm chambers 108,110 are coupled to internal conduits 112,114,116,118 which can be filled with fluid and then closed with a slug or screw threaded member which is adjustable so as to vary the pressure in the chamber. With this arrangement the system can be calibrated at the beginning of each measurement procedure.

In operation the system operates substantially identically to the above described embodiment. The inlet fluid is pumped through the filtering unit and the inlet conduit is fed through the removable cassette portion 14 and in particular through inlet 94. The inlet pressure is measured by pressure sensor 113. Similarly the filter output is coupled to the cassette via internal conduits 102 and 106 and the filter outlet pressure is measured by sensor 115 coupled to diaphragm 104. In this way the pressure across the membrane, the transmembrane pressure is measured and the measured pressure can be averaged and used to control the flow through the filter unit so to optimise the transmembrane pressure and hence filtration. The transmembrane pressure signal can be used to control the pressure switch or valve can indicated above. An advantage of this arrangement is that there is no requirement for solenoid valves purging because there is no contamination of the measurement line.

The transmembrane pressure filtration and separation system hereinbefore described has particular relevance in Downstream Processing, which is the isolation of cells and products from fermentation broths and other high value liquids. The use of a cross-flow mechanism in combination with low shear faces and low transmembrane pressures, results in reduced stress on sensitive cells, genetically engineered or otherwise, avoiding or minimising cell lysis and therefore avoiding the release of enzymes that may have an inhibiting or destructive effect on the final products.

The Bio-Flo Downstream processing unit offers the operator full control over filtration or separation processes by means of a built-in solid state flow and pressure control mechanism and the cassette and diaphragm arrangement allows the system to be used under full containment conditions. The downstream processing unit is similar to the arrangement hereinbefore described with reference to Figs. 1 to 5, and includes a microprocessor which monitors pressure within the fluidlines and which switches on a back-flush pumping system once the pressure within the fluidlines exceeds a preset maximum level. This is best explained with reference to Fig. 7 which is a graph of pressure against time and shows that the back-wash procedure occurs when the pressure reaches 1.2 bar, for example. The period of back flushing is dependent on the material being processed and can be programmed by the operator. Because only one filter, or a set of filters may be back-washed at any one time, the filtration process is an ongoing process avoiding cell settlement in the fluidlines and filters. Also, the use of autoclavable filters and fluidlines allow for aseptic conditions when processing, for example, sterile fluids.

It will be appreciated that various modifications may be made to the apparatus as hereinbefore described without departing from the scope of the invention.

## Claims

1. A membrane filtration device comprising filter means (60) having an inlet (58) for receiving an inlet fluid to be filtered and an outlet (74) for receiving the outlet fluid from said filter means (60), first pressure monitoring means (65) associated with the inlet (58) for measuring the inlet pressure to said filter means (60), second pressure monitoring means (84) associated with the outlet (74) for measuring the pressure of the outlet fluid, means for comparing the inlet and outlet pressures measured and flow control means (67) coupled to said first and second pressure monitoring means (65, 84) to provide a comparison signal, the flow control means (67) being responsive to the comparison signal to control the flow of fluid through said filter means (60), characterised in that the device further comprises an inlet bleed conduit (62) for fluid communication between the inlet (58) and outlet (74), and wherein the control of the flow of fluid through the filter means (60) is achieved by varying the flow of inlet fluid through the inlet bleed conduit (62), to optimise control of the transmembrane pressure and filtration.

2. The device as claimed in claim 1 wherein the fluid flow control means is a pressure switch (67) located in the inlet bleed conduit (62).

3. The device as claimed in claim 1 wherein the fluid flow control means is a pressure switch (80) located in an outlet bleed conduit (78) or is a valve for restricting flow in the inlet or outlet bleed conduit.

4. The device as claimed in any preceding claim wherein the pressure monitoring means (65, 84) are disposed in the inlet bleed conduit (62) and in an outlet bleed conduit (78), respectively, so that the fluid flows past said means (65, 84).

5. The device as claimed in any one of claims 1 to 3 wherein the pressure monitoring means are diaphragm pressure sensors so that there is no contact between the fluid being treated and the pressure monitoring means.

6. The device as claimed in any preceding claim wherein the first and second pressure monitoring means (65, 84) are coupled to first and second solenoid valves (67, 80) respectively, the outputs of which are combined within a single bleed conduit (62, 78), the valves being actuatable to purge fluid from the conduit.

7. The device as claimed in any preceding claim in combination with a fluid flow control apparatus (10) which includes a fixed portion (12) and a removable portion (14), the fixed portion (12) having pressure monitoring means (67, 84) and a pressure switch disposed therein and which are coupled to internal conduits within the fixed portion to facilitate flow-through of the fluid being monitored.

8. The combination as claimed in claim 7 wherein the removable portion (14) contains internal conduits which terminate in ports (23a, 23b) which mate with corresponding ports (22a, 22b) in the fixed portion (12) so as to form aseptic ports when the removable portion (14) is inserted into the fixed portion (12).

9. The combination as claimed in claim 7 wherein the removable portion (14) contains at least two diaphragms (98, 104) each of which is disposed in the surface of the removable portion (14), one diaphragm (104) being arranged to receive, through internal conduits, fluid from the filter means (60) and the output of which is fed to a valve, the other diaphragm (98) being arranged to receive fluid from a pump and to return this fluid to the filter means (60).

10. The combination as claimed in claim 9 wherein disposed in the fixed portion (12) are corresponding diaphragms (108, 110) such that when the removable portion (14) is inserted into the fixed portion (12), the diaphragms register with each other, the diaphragms (108, 110) on the fixed portion being coupled to respective pressure gauges for monitoring the filter inlet and outlet pressures and coupled to internal conduits which are filled with fluid to calibrate the pressure sensors.

11. The device as claimed in claim 1 including processing means coupled to the inlet and outlet for measuring pressure therein and back-flush pumping means coupled to said processing means which is actuatable via said processing means to back-flush the system when the measured pressure exceeds a preset level.

## Patentansprüche

1. Membranfiltervorrichtung, umfassend ein Filtermittel (60) mit einem Einlaß (58) zur Aufnahme eines einströmenden, zu filternden Fluids und einem Auslaß (74) zur Aufnahme des von dem Filtermittel (60) ausströmenden Fluids, ein erstes Druckaufzeichnungsmittel (65), das dem Einlaß (58) zur Messung des Eingangsdruckes zu dem Filtermittel (60) zugeordnet ist, ein zweites Druckaufzeichnungsmittel (84), das dem Auslaß (74) zur Messung des Druckes des ausströmenden Fluids zugeordnet ist, ein Mittel zum Vergleich der gemessenen Einlaß- und Auslaßdrücke und ein Durchflußsteuermittel (67), das an das erste und zweite Druckaufzeichnungsmittel (65, 84) gekoppelt ist, um ein Vergleichssignal zu liefern, wobei das Durchflußsteuermittel (67) auf das Vergleichssignal zur Steuerung des Fluidflusses durch das Filtermittel (60) anspricht, dadurch gekennzeichnet, daß die Vorrichtung ferner eine Einlaßableitung (62) zur Fluidverbindung zwischen dem Einlaß (58) und dem Auslaß (74) umfaßt und wobei die Steuerung des Fluidflusses durch das Filtermittel (60) durch Veränderung des Durchflusses des einströmenden Fluids durch die Einlaßableitung (62) erzielt wird, um die Steuerung des transmembranen Druckes und die Filtrierung zu optimieren.

2. Vorrichtung nach Anspruch 1, wobei das Fluiddurchflußsteuermittel ein Druckschalter (67) ist, der in der Einlaßableitung (62) angeordnet ist.

3. Vorrichtung nach Anspruch 1, wobei das Fluiddurchflußsteuermittel ein Druckschalter (80) ist, der in einer Auslaßableitung (78) angeordnet ist, oder ein Ventil zur Begrenzung des Durchflusses in der Einlaß- oder Auslaßableitung ist.

4. Vorrichtung nach einem der vorangehenden Ansprüche, wobei die Druckaufzeichnungsmittel (65, 84) in der Einlaßableitung (62) bzw. in einer Auslaßableitung (78) angeordnet sind, so daß das Fluid über diese Mittel (65, 84) strömt.

5. Vorrichtung nach einem der Ansprüche 1 bis 3, wobei die Druckaufzeichnungsmittel Membrandrucksensoren sind, so daß kein Kontakt zwischen dem behandelten Fluid und den Druckaufzeichnungsmitteln besteht.

6. Vorrichtung nach einem der vorangehenden Ansprüche, wobei das erste und zweite Druckaufzeichnungsmittel (65, 84) an ein erstes bzw. zweites Solenoidventil (67, 80) gekoppelt ist, deren Ausgänge in einer einzigen Ableitung (62, 78) vereint sind, wobei die Ventile betätigbar sind, um Fluid aus der Leitung zu spülen.

7. Vorrichtung nach einem der vorangehenden Ansprüche in Kombination mit einer Fluiddurchflußsteuervorrichtung (10), welche einen feststehenden Teil (12) und einen entfernbaren Teil (14) umfaßt, wobei in dem feststehenden Teil (12) Druckaufzeichnungsmittel (67, 84) und ein Druckschalter angeordnet sind, die in dem feststehenden Teil mit Innenleitungen verbunden sind, um den Durchfluß des aufgezeichneten Fluids zu erleichtern.

8. Kombination nach Anspruch 7, wobei der entfernbare Teil (14) Innenleitungen enthält, die in Öffnungen (23a, 23b) münden, welche zu den entsprechenden Öffnungen (22a, 22b) in dem feststehenden Teil (12) passen, so daß sterile Öffnungen entstehen, wenn der entfernbare Teil (14) in dem feststehenden Teil (12) eingesetzt ist.

9. Kombination nach Anspruch 7, wobei der entfernbare Teil (14) mindestens zwei Membranen (98, 104) enthält, von welchen jede in der Oberfläche des entfernbaren Teils (14) angeordnet ist, wobei eine Membran (104) zur Aufnahme durch Innenleitungen von Fluid von dem Filtermittel (60) angeordnet ist und ihr Ausgang einem Ventil zugeleitet wird, und die andere Membran (98) zur Aufnahme von Fluid von einer Pumpe und zur Rückleitung dieses Fluids zu dem Filtermittel (60) angeordnet ist.

10. Kombination nach Anspruch 9, wobei in dem feststehenden Teil (12) entsprechende Membranen (108, 110) angeordnet sind, so daß, wenn der entfernbare Teil (14) in den feststehenden Teil (12) eingesetzt ist, die Membrane miteinander ausgerichtet sind, wobei die Membranen (108, 110) auf dem feststehenden Teil mit entsprechenden Druckmessern zur Aufzeichnung des Filtereinlaß- und -auslaßdruckes verbunden sind und mit Innenleitungen verbunden sind, die mit Fluid zur Kalibrierung der Drucksensoren gefüllt sind.

11. Vorrichtung nach Anspruch 1, umfassend ein Verarbeitungsmittel, das mit dem Einlaß und Auslaß zur Messung des enthaltenen Druckes verbunden ist, und ein Rückspülpumpmittel, das mit dem Verarbeitungsmittel verbunden und über das Verarbeitungsmittel betätigbar ist, um das System zurückzuspülen, wenn der gemessene Druck einen vorgegebenen Wert übersteigt.

## Revendications

1. Dispositif de filtration à membrane comprenant un filtre (60) ayant une entrée (58) pour recevoir un fluide d'entrée à filtrer et une sortie (74) pour recevoir le fluide de sortie dudit filtre (60), des premiers moyens de contrôle de pression (65) associés à l'entrée (58) pour mesurer la pression d'entrée audit filtre (60), des deuxièmes moyens de controle de pression (84) associés à la sortie (74) pour mesurer la pression du fluide de sortie, des moyens de comparaison des pressions mesurées d'entrée et de sortie et un élément de commande d'écoulement (67) relié aux dits premiers et deuxièmes moyens de contrôle de pression (65,84) fournissant un signal de comparaison, l'élément de commande d'écoulement (67) répondant au signal de comparaison de manière à régler le débit de fluide à travers ledit filtre (60), caractérisé en ce que le dispositif comprend en outre un conduit de dérivation d'entrée (62) pour une communication de fluide entre l'entrée (58) et la sortie (74), et en ce que le réglage du débit de fluide à travers le filtre (60) est effectué par modification du débit de fluide d'entrée passant dans le conduit de dérivation d'entrée (62), afin d'optimiser le réglage de la pression transmembrane et de la filtration.

2. Dispositif suivant la revendication 1, dans lequel l'élément de commande d'écoulement de fluide est un manocontact (67) placé dans le conduit de dérivation d'entrée (62).

3. Dispositif suivant la revendication 1, dans lequel l'élément de commande d'écoulement de fluide est un manocontact (80) placé dans un conduit de dérivation de sortie (78), ou une vanne de limitation du débit dans le conduit de dérivation d'entrée ou de sortie.

4. Dispositif suivant une quelconque des revendications précédentes, dans lequel les moyens de contrôle de pression (65,84) sont disposés dans le conduit de dérivation d'entrée (62) et dans un conduit de dérivation de sortie (78), respectivement, de sorte que le fluide s'écoule devant lesdits moyens (65,84).

5. Dispositif suivant une quelconque des revendications 1 à 3, dans lequel les moyens de contrôle de pression sont des capteurs de pression à membrane de sorte qu'il n'y a pas de contact entre le fluide à traiter et les moyens de contrôle de pression.

6. Dispositif suivant une quelconque des revendications précédentes, dans lequel les premiers et deuxièmes moyens de contrôle de pression (65,84) sont reliés à une première et une deuxième électrovannes (67,80), respectivement, dont les sorties sont combinées dans un unique conduit de dérivation (62,78), les vannes étant actionnables pour purger le fluide du conduit.

7. Dispositif suivant une quelconque des revendications précédentes, en combinaison avec un appareil de commande d'écoulement de fluide (10) qui comprend une partie fixe (12) et une partie amovible (14), la partie fixe (12) comportant des moyens de contrôle de pression (67,84) et un manocontact disposés dans cette partie et reliés à des conduits internes prévus dans la partie fixe pour faciliter l'écoulement traversant du fluide à contrôler.

8. Combinaison suivant la revendication 7, dans laquelle la partie amovible (14) contient des conduits internes se terminant à des orifices (23a,23b) qui s'accouplent à des orifices correspondants (22a,22b) de la partie fixe (12) de façon à former des orifices aseptiques lorsque la partie amovible (14) est insérée dans la partie fixe (12).

9. Combinaison suivant la revendication 7, dans laquelle la partie amovible (14) contient au moins deux membranes (98,104) dont chacune est disposée dans la surface de la partie amovible (14), une membrane (104) étant agencée pour recevoir, par l'intermédiaire de conduits internes, le fluide venant du filtre (60) et sa sortie étant envoyée à une vanne, l'autre membrane (98) étant agencée pour recevoir le fluide venant d'une pompe et pour renvoyer ce fluide au filtre (60).

10. Combinaison suivant la revendication 9, dans laquelle des membranes correspondantes (108,110) sont disposées dans la partie fixe (12) de sorte que, lorsque la partie amovible (14) est insérée dans la partie fixe (12), les membranes s'alignent mutuellement, les membranes (108,110) de la partie fixe étant reliées à des manomètres respectifs pour mesurer les pressions d'entrée et de sortie du filtre et étant reliées à des conduits internes qui sont remplis de fluide pour le calibrage des capteurs de pression.

11. Dispositif suivant la revendication 1, comprenant des moyens de traitement reliés à l'entrée et à la sortie pour y mesurer la pression, et des moyens de pompage à contre-courant reliés auxdits moyens de traitement et actionnables par lesdits moyens de traitement pour laver le système à contre-courant lorsque la pression mesurée dépasse une valeur prédéterminée.
